(19) Europäisches Patentamt / European Patent Office / Office européen des brevets

(11) EP 3 738 962 B1

(12) **EUROPEAN PATENT SPECIFICATION**

(45) Date of publication and mention of the grant of the patent:
**02.08.2023 Bulletin 2023/31**

(21) Application number: **19738948.9**

(22) Date of filing: **11.01.2019**

(51) International Patent Classification (IPC):
***C07D 409/12*** (2006.01) ***A61K 31/416*** (2006.01)
***A61P 37/08*** (2006.01) ***A61P 11/06*** (2006.01)
***A61P 11/08*** (2006.01) ***A61P 29/00*** (2006.01)

(52) Cooperative Patent Classification (CPC):
**A61P 7/02; A61K 31/416; A61P 1/00; A61P 11/00; A61P 11/06; A61P 11/08; A61P 15/00; A61P 17/00; A61P 19/00; A61P 19/02; A61P 19/06; A61P 19/10; A61P 27/02; A61P 29/00; A61P 31/00;** (Cont.)

(86) International application number:
**PCT/CN2019/071315**

(87) International publication number:
**WO 2019/137463 (18.07.2019 Gazette 2019/29)**

(54) **SALTS OF A COMPOUND AND USE THEREOF**

SALZE EINER VERBINDUNG UND DEREN VERWENDUNG

SELS DE COMPOSÉ ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.01.2018 CN 201810032410**

(43) Date of publication of application:
**18.11.2020 Bulletin 2020/47**

(73) Proprietor: **Medspring International Limited**
**Hong Kong (CN)**

(72) Inventor: **ZHAO, Hai Feng**
**Hong Kong (CN)**

(74) Representative: **Long, Giorgio**
**Jacobacci & Partners S.p.A.**
**Via Senato, 8**
**20121 Milano (IT)**

(56) References cited:
**EP-A1- 2 401 270 CN-A- 102 388 039**

- **PROCOPIOU, P.A. et al.: "Lead Optimisation of the N1 Substituent of a Novel Series of Indazole Arylsulfonamides as CCR4 Antagonists and Identification of a Candidate for Clinical Investigation", Bioorganic & Medicinal Chemistry Letters, vol. 22, no. 8, 7 March 2012 (2012-03-07), pages 2730-2733, XP055625236, ISSN: 0960-894X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
**A61P 31/04; A61P 31/10; A61P 31/12; A61P 31/16; A61P 35/00; A61P 37/08; C07D 409/12**

## Description

The present application claims priority to the Chinese patent application No. 201810032410.2, entitled "Compound, Preparation Method therefor, and Use thereof" and filed January 12, 2018.

## TECHNICAL FIELD OF THE INVENTION

The present invention relates to pharmaceutically acceptable salts of a compound and to pharmaceutical compositions containing the salts and their application in the preparation of a pharmaceutical drug, which can be used for the treatment of CCR4- mediated diseases.

## BACKGROUND

CCR4 (chemokine receptor 4) was first discovered by Christine A. Power in 1995 (Christine AP et al. J. Biol. Chem. 1995, 270 (8): 19495-19500). It belongs to the chemokine receptor (CCR) family and is a seven-time transmembrane G-protein coupled receptor. It has two naturally existing specific ligands: MDC (macrophage derived chemokine) and TARC (thymus and activation regulated chemokine) (Sadatoshi Maeda et al., Veterinary Immunology and Immunology 2002 (90): 145-154). A newly discovered chemokine like factor 1 (CKLF1) is also one of its ligands (Han WL et al., Biochem J. 2001, 357 (Pt1): 127-135).

CCR4 can be expressed in peripheral blood leukocytes, thymocytes, basophils, monocytes, macrophages, platelets, IL-activated NK cells, spleen, and brain, etc., and plays an important role in many diseases. For example, upon onset of human allergic dermatitis (AD), the expression of CCR4 on CD4+T cells is increased in peripheral blood monocytes (PBMCs), and the serum TARC level is also increased accordingly. This suggests that the chemotaxis of CCR4 expression in cells is induced by TARC and selectively causes Th2 cells to migrate to damaged skin in the allergic dermatitis. Drugs used to treat allergic dermatitis mainly include antihistamines and bronchodilators, which nonetheless can only improve symptoms, but have no effects on the development of the disease. In addition, corticosteroids also have certain effects on the allergic dermatitis, but there are potential safety risks. Studies have shown that antagonism against MDC or TARC can reduce the aggregation of T cells in inflammatory sites. CCR4 antagonists may be effective in the treatment of allergic inflammations, which include, but are not limited to, allergic rhinitis and allergic dermatitis.

CCR4 has been found to be associated with lung diseases such as chronic obstructive pneumonia, chronic bronchitis, and asthma. CCR4 can be limited to being expressed in cells involved in asthma response, and thus is considered to be a good target for the treatment of asthma. Therefore, the development of CCR4 antagonists has a promising application prospect.

CN101370793A discloses compounds having the following formula and their use as CCR4 antagonists, including their application in the CCR4 mediated diseases

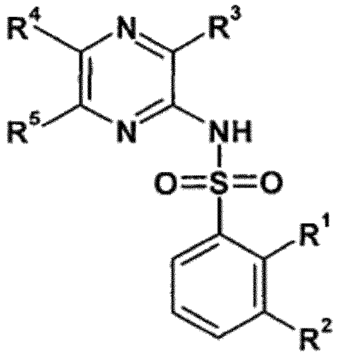

,

wherein the various substituent groups are disclosed in the patent application.

CN102388039A discloses a class of indazole compounds and their use as CCR4 antagonists, including their application in the treatment that is related to CCR4 antagonists

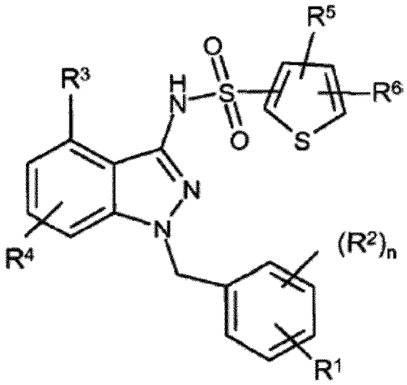

,

wherein the various substituent groups are disclosed in the patent application.

In the field, better CCR4 inhibitors are still needed, which are characterized by having, for example, a better CCR4 inhibitory activity, a higher solubility in water or other solvents, or a higher bioavailability.

**SUMMARY OF THE INVENTION**

The invention is defined in the appended claims. The subject-matter not covered by the claims is for illustrative purposes only.

Provided in this disclosure is a compound of formula I, and its pharmaceutically acceptable salts, solvates, or prodrugs.

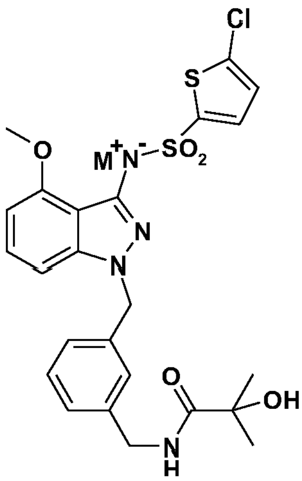

I

Herein, M can be a monovalent alkali metal. For example, M can be Li, Na, K, Rb, or Cs. According to the invention, M is Na or K.

In one aspect of the invention, the compound of formula I is

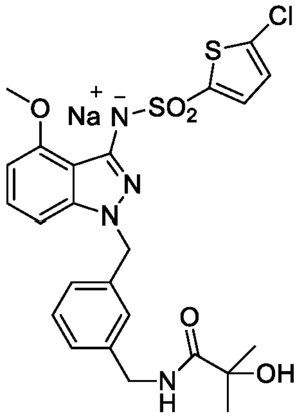

II

In another aspect of the invention, the compound having formula I is

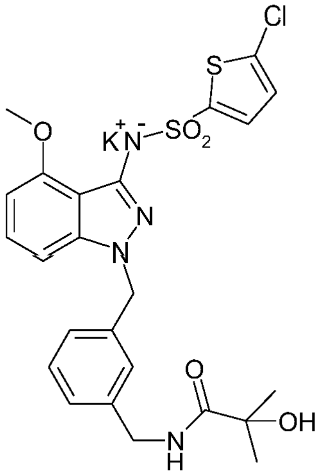

III

In this present disclosure, the suitable pharmaceutically acceptable salt of the compound of formula I (not part of the invention) can be, for example, an acid addition salt of the compound of formula I, such as an acid addition salt formed with an inorganic or organic acid (e.g. hydrochloric acid, hydrobromic acid, sulfuric acid, trifluoroacetic acid, citric acid, or maleic acid), or can be an ammonium salt, or a salt formed with organic bases (e.g. methylamine, dimethylamine, trimethylamine, piperidine, morpholine, or tri-(2-hydroxyethyl) amine).

In this present disclosure, the suitable pharmaceutically acceptable solvate for the compound of formula I (not part of the invention) can be, for example, a hydrate, such as a hemihydrate, a monohydrate, a dihydrate, a trihydrate, or a combination thereof.

In this present disclosure, the compound can be administered in the form of prodrugs (not part of the invention).

Herein, the term "prodrug" is referred to as a compound which is decomposed or cracked in human body or animal body to thereby release the compound provided in this present disclosure. Examples of prodrugs include *in vivo* cleavable ester derivatives and *in vivo* cleavable amide derivatives. The ester derivatives can be formed on the hydroxyl groups of the compound of formula I, and the amide derivatives can be formed on the amino groups of the compound of formula I.

A pharmaceutically acceptable prodrug of the compound of formula I (not part of the invention) can be, for example, an ester or ether that is cleavable or decomposable in a body. The *in vivo* cleavable esters or ethers of the compound of formula I containing hydroxyl groups can be pharmaceutically acceptable esters or ethers, such as esters or ethers that can be cleaved in humans and animals to produce parental hydroxyl compounds. With regard to hydroxyl groups, suitable pharmaceutically acceptable ester-forming groups can include inorganic esters such as phosphate esters (including aminophosphocyclic esters). Furether with regard to the hydroxyl groups, suitable pharmaceutically acceptable ester-forming groups can also include (1-10C) alkanoyl groups (e.g. acetyl group, benzoyl group, phenylacetyl group and substituted benzoyl group, and phenylacetyl group), and (1-10C) alkoxycarbonyl groups (e.g. ethoxycarbonyl

group, *N, N*-[di-(1-4C) alkyl] carbamoyl group, 2-dialkylaminoacetyl group, and 2-carboxyacetyl group). Examples of the substituents on the phenylacetyl group and benzoyl group can include aminomethyl group, n-alkylaminomethyl group, *N*,*N*-dialkylaminomethyl group, morpholinomethyl group, piperazine-1-ylmethyl group, and 4-(1-4C) alkyl piperazine-1-ylmethyl group. Furether with regard to the hydroxyl groups, suitable pharmaceutically acceptable ether-forming groups can include $\alpha$-acyloxyalkyl groups such as acetoxymethyl group and pivaloyloxymethyl group.

In this present disclosure, the compound of formula I or its pharmaceutically acceptable salts, solvates, or prodrugs have an CCR4 inhibitor activity. As such, the compound of formula I or its pharmaceutically acceptable salts, solvates, or prodrugs can be used for the treatment of CCR4-mediated diseases, or can be used for the preparation of medicines for the treatment of CCR4-mediated diseases. Specifically, the compound of formula I provided in the present disclosure, or its pharmaceutically acceptable salts, solvates or prodrugs can be used to treat diseases in which the regulation of CCR4 (e.g., inhibiting the activity of CCR4) is beneficial to patients.

In one aspect of the present invention, a pharmaceutical composition is provided, which comprises the compound of formula II or III described above and a pharmaceutically acceptable excipient mixed therewith, such as a diluent or a carrier. The pharmaceutical composition can be used for the treatment of CCR4-mediated diseases.

The pharmaceutical composition provided in the present disclosure can include a conventional pharmaceutical excipient that is well known in the art, and can be obtained by a conventional method. For example, an excipient included in a pharmaceutical composition that is intended for oral administration can comprise one or more of a colorant, a sweetener, a flavoring agent, and/or a preservative, etc.

The pharmaceutical composition provided in the present disclosure can be used in a form that is suitable for oral administration (e.g. tablets, pastilles, hard or soft capsules, water or oil suspensions, emulsions, dispersible powder or granules, syrup, or elixir), in a form suitable for topical administration (e.g. cream, ointment, gel, water or oily solution or suspension), in a form suitable for inhalation administration (e.g. finely dispersed powder or liquid aerosol), in a form suitable for spray (e.g. fine dispersible powder), or in a form suitable for parenteral administration (e.g., sterile water or oily solution for intravenous, subcutaneous, intraperitoneal or intramuscular administration, or suppositories for rectal administration).

The amount of the active ingredient that is combined with one or more excipients to form a single dosage form can change based on needs, depending on the subject receiving the treatment and the specific route of administration. For example, the dosage form intended for oral administration to human subjects generally can contain, e.g. 0.1 mg-1 g (more preferably 1-250 mg, e.g., 1-100 mg) of the active ingredient mixed with an appropriate and convenient amount of excipients, which can account for about 5-98% of the total weight of the composition.

The dosage size of the compound of formula II or III for therapeutic or preventive purposes typically can vary according to well-known medical principles, depending on the nature and severity of the disease, the age and sex of an animal or a patient, and the route of administration.

In one aspect of the present disclosure, the compounds of formula II or III as described above are provided for their use in the treatment (or prevention) of a CCR4-mediated disease. According to the invention, the CCR4-mediated diseases are asthma, COPD and rhinitis.

In another aspect of the present disclosure, the compounds of formula II or III as described above are provided for use in the preparation of drugs or medicines for the treatment (or prevention) of CCR4 mediated diseases. According to the invention, the CCR4-mediated diseases are asthma, COPD and rhinitis.

The CCR4 mediated diseases can be diseases and conditions associated with inflammation or infection. The CCR4 mediated diseases can include:

(1) respiratory diseases, such as obstructive diseases of airways including: asthma, which includes bronchial, allergic, intrinsic, extrinsic, exercise-induced, drug-induced (including aspirin and NSAID-induced), and dust-induced asthma, and intermittent and persistent asthma; chronic obstructive pulmonary disease (COPD); bronchitis, which includes infectious and eosinophilic bronchitis; emphysema; bronchiectasis; cystic fibrosis; hypersensitivity pneumonitis; lung fibrosis, which includes cryptogenic fibrosing alveolitis, idiopathic interstitial pneumonias, and fibrosis complicating anti-neoplastic therapy and chronic infection; vasculitic and thrombotic disorders of the lung vasculature, and pulmonary hypertension; rhinitis, which includes acute and chronic rhinitis, and includes rhinitis medicamentosa, and vasomotor rhinitis, perennial and seasonal allergic rhinitis including rhinitis nervosa; acute viral infection including common cold, and infection cased by respiratory syncytial virus, influenza, coronavirus and adenovirus;

(2) inflammation of bone and joints, such as arthritides associated with or including osteoarthritis or osteoarthrosis; cervical and lumbar spondylitis, low back pain, and neck pain; osteoporosis; rheumatoid arthritis; acute and chronic synovitis, including urate gout, calcium pyrophosphate deposition disease, and calcium apatite-related tendon, bursal and synovial inflammation; inflammatory myopathies, including dermatomyositits and polymyositis; polymalgia rheumatica; juvenile arthritis including idiopathic inflammatory arthritides of whatever joint distribution and associated syndromes, and rheumatic fever and its systemic complications; and drug-induced arthalgias, tendonititides, and myopathies;

(3) psoriasis and inflammatory dermatosis, such as dermatitis, eczema, atopic dermatitis, allergic contact dermatitis, rubella and pruritus;

(4) blepharitis and conjunctivitis, including perennial allergic conjunctivitis or spring allergic conjunctivitis; iritis; anterior and posterior uveitis; choroiditis; autoimmune eye diseases; degenerative or inflammatory disorders affecting the retina; ophthalmitis including sympathetic ophthalmitis; sarcoidosis; and eye infections, including viral, fungal, and bacterial infections; and

(5) other diseases associated with CCR4, such as gastrointestinal inflammation, genitourinary inflammation, and immune system inflammation, etc.

In yet another aspect of the present disclosure, a method for preparing a compound of formula I is provided.

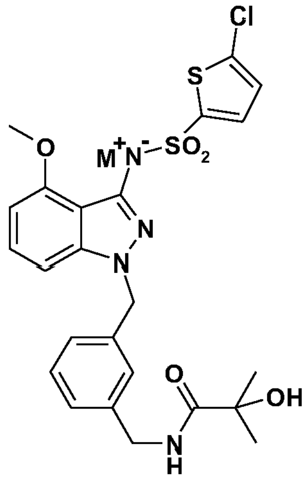

I

Herein, M can be a monovalent alkali metal, such as Li, Na, K, Rb or Cs, with Na or K being the alkali metals of the invention.

The method comprises: stir-mixing the compound 6 of the formula:

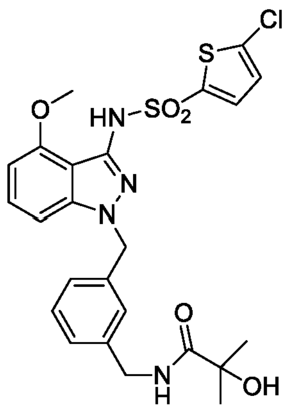

6

with a strong alkali of the monovalent alkali metal M in an organic solvent, which is then filtered and dried. Preferably, the molar ratio of the compound 6 to the strong alkali of the monovalent alkali metal M is approximately 1:0.5-2. More preferably, the molar ratio of the compound 6 to the strong alkali of the monovalent alkali metal M is approximately 1:1.

The reaction can be carried out in a suitable organic solvent. For example, the organic solvent can be an ester solvent, an aromatic hydrocarbon solvent, an ether solvent, an alcohol solvent, or another water-soluble polar solvent: the ester solvent can be methyl acetate, ethyl acetate, propyl acetate or butyl acetate; the aromatic solvent can be benzene, toluene, ethylbenzene, isopropyl benzene or xylene; the ether solvent is ether, isopropyl ether, methyl tert butyl ether, tetrahydrofuran, 1,4-dioxane, ethylene glycol dimethyl ether, or diethylene glycol dimethyl ether; the alcohol solvent can be methanol, ethanol, propanol, n-butanol or ethylene glycol; the another water-soluble polar solvents can

be acetone, butanone, N, N-dimethylformamide, N, N-dimethylacetamide or dimethyl sulfoxide. The strong alkali of the monovalent alkali metal M can be a hydride of M, such as NaH or KH. The strong alkali of the monovalent alkali metal M can also be an alkoxide of M, such as sodium tert-butoxide or potassium tert-butoxide. The strong alkali can also be an oxide, a hydroxide or a bicarbonate of the alkali metal M. In another aspect of the present invention, the method comprises: dissolving the compound 6 in an organic solvent, adding MH or an alkoxide of M, stirring-mixing for a reaction (e.g., at a temperature of 15°C-35 °C for 1-3.5 hours), and filtering and drying. Preferably, the molar ratio of the compound 6 to the MH or the alkoxide of M is 1:0.5-2, and more preferably, the molar ratio of the compound 6 to the MH or the alkoxide of M is about 1: 1.

In yet another aspect of the present disclosure, a method for preparing a compound of formula II shown below is provided.

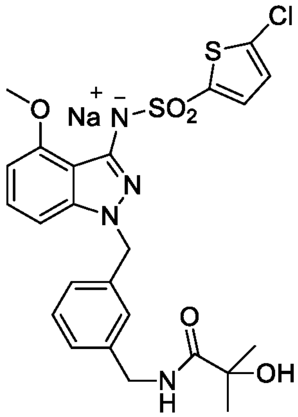

II

Herein, the method comprises: dissolving the compound 6 in an organic solvent, adding NaH, stirring-mixing for a reaction (e.g., at a temperature of 15°C-35°C for 1-3.5 hours), and filtering and drying. Preferably, the molar ratio of the compound 6 to the NaH is 1:0.5-2, and more preferably is about 1:1.

In yet another aspect of the present disclosure, a method for preparing a compound of formula III shown below is provided.

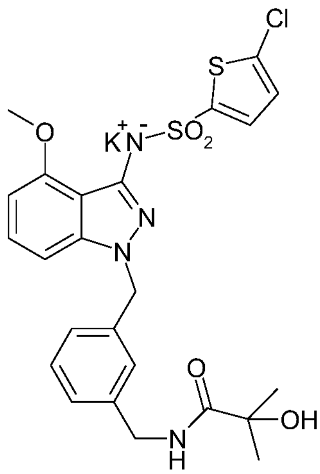

III

Herein, the method comprises: dissolving the compound 6 in an organic solvent, adding KH, stirring-mixing for a reaction (e.g., at a temperature of 15°C-35°C for 1-3.5 hours), and filtering and drying. Preferably, the molar ratio of the compound 6 to the KH is 1:0.5-2, and more preferably, is about 1:1.

In yet another aspect of the present disclosure, a method for preparing a compound of formula III shown below is provided.

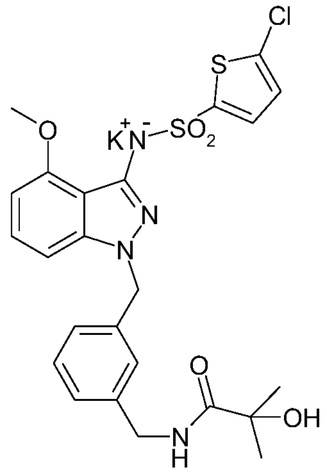

III

Herein, the method comprises: dissolving the compound 6 in an organic solvent, adding potassium tert butyl alcohol, stirring-mixing for a reaction (e.g., at a temperature of 15°C-35°C for 1-3.5 hours), and filtering and drying. Preferably, the molar ratio of the compound 6 to the potassium tert-butoxide is 1:0.5-2, and more preferably, is about 1: 1.

Currently existing CCR4 inhibitors have an issue of low solubility, which increases the difficulty in drug preparation, affects the release of active ingredients, causes low bioavailability, and fails to reach an expected blood drug concentration *in vivo* at a maximum safe dose, thereby affecting the efficacy of the drugs. The compound of formula I, especially the compounds of formula II and formula III are better CCR4 inhibitors, which have advantages over the prior art, including:

1. Significantly enhanced solubility. Compared with compound 6, compound II and compound III have significantly enhanced solubility under the same conditions. For example, in an aqueous solution at room temperature, the solubility of compounds of formula II and formula III is 500 or 40 mg/ml, whereas the solubility of compound 6 is 0.02 mg/ml under the same conditions, thus the solubility of compounds II and III is 2000-25000 times higher than that of compound 6.
2. Better bioavailability. Based on a standard CaCO-2 *in vitro* membrane permeability evaluation, results showed that the $P_{app}$ (apparent permeability coefficient) of compound 6 is $0.13 \times 10^{-6}$, indicating that compound 6 is a difficult-to-absorb compound, whereas the $P_{app}$ of compounds of formula II and formula III is $68.82\text{-}101.33 \times 10^{-6}$ under the same experimental conditions, which is 530-780 times higher in terms of the permeability, indicating that compounds of formula II and formula III are easy-to-absorb compounds.
3. Better *in vitro* and *in vivo* activity. Radiolabeling experiments show that the IC50 of the compounds of formula II and formula III are $2.26 \times 10^{-9}$ mol/L - $7.47 \times 10^{-10}$ mol / L, which are 6 and 210 times higher than that of compound 6 under the same experimental conditions.

## DETAILED DESCRIPTION OF THE INVENTION

In the following, with combination of embodiments, description of the substantive contents and the beneficial effects of the present invention is further provided. It is noted that the embodiments serve only to illustrate, and are not intended to limit the scope of, the present invention.

Embodiment 1 Preparation of N-[(3-{[3-{[(5-chloro-2-thienyl)sulfonyl]amino}-4-(methoxy)- 1H-indazole-1-yl]methyl}phenyl)methyl]-2-hydroxy-2-methyl propionamide.

Preparation of compound 6 having the following formula: N-[(3-{[3-{[(5-chloro-2-thienyl)sulfonyl]amino}-4-(methoxy)-1H-indazole-1-yl]methyl}phenyl) methyl]-2-hydroxy-2-methyl propionamide is provided.

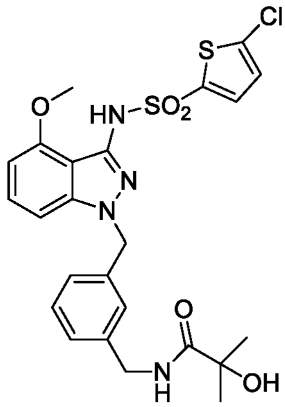

6

Step 1 Preparation of an intermediate compound 1 (show below): 4-(methoxy)-1H-indazole-3 -amine.

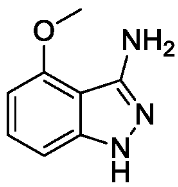

1

6-fluoro-2-methoxybenzonitrile (50g, 0.33 mol) and hydrazine hydrate (50g, 0.99mol) were dissolved in 400 ml n-butanol, and refluxed for 20h under the protection of $N_2$. After the solution was cooled down, 400ml water was added, the reaction was stirred to mix evenly, the organic layer is separated, and the solid precipitated from the aqueous phase was collected. The organic layer solvent was removed by vacuum distillation, and the residues were mixed with the aqueous phase and the solid precipitated from the aqueous phase, and the mixture was extracted with ethyl acetate (3 × 400ml). The organic layer was washed with water (2 × 400ml) and dried with anhydrous magnesium sulfate. The gray white solid (46.32g, yield 85.8%) was obtained by vacuum drying after solvent removal by vacuum distillation. MS ESI+ 164(M+H)$^+$.

Step 2 Preparation of an intermediate compound 2 (show below): 3-{[3-amino-4-(methoxy)-1H-indazole-1-yl]methyl}benzonitrile.

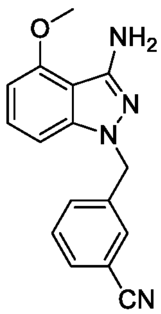

2

Potassium hydroxide (9g, 160.7mmol) and DMSO (200ml) were mixed and stirred evenly. Then 4-(methoxy)-1H-indazole-3-amine (10.48g, 64.3mmol) was added, and the solution turned dark purple. After stirring for 10 min at room temperature, 3-(chloromethyl)benzonitrile (11.8g, 64.3mmol) was added and stirred to mix for 2h. 500ml water was then added to the reaction solution, and the reaction changes into an emulsion, which further underwent dichloromethane extraction (3 × 400ml), water washing with water (2 × 400ml), anhydrous magnesium sulfate drying, column chromatography (dichloromethane/ethyl acetate = 5/1), solvent removal by vacuum distillation, and vacuum drying. A light yellow solid (13.22g, yield 73.9%) was obtained. MS ESI+ 279$(M+H)^+$.

Step 3 Preparation of an intermediate compound 3 (shown below): 5-chloro-N-{1-[(3-cyanophenyl)methyl]-4-(methoxy)-1H-indazole-3-yl}-2-thiophene sulphonamide.

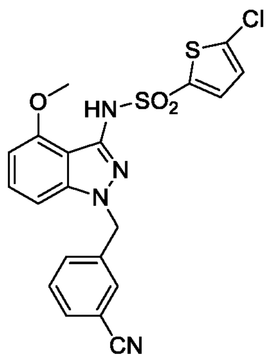

3

Pyridine (12ml) solution of 5-chloro-2-thiophenesulfonyl chloride (7.96g, 36.7mmol) was dropped into a two-mouthed bottle containing 3-{[3-amino-4-(methoxy)-1H-indazole-1-yl]methyl}benzonitrile (10.2g, 36.7mmol) at room temperature and under a nitrogen atmosphere. The reaction system turns dark red. One hour later, the reaction solution was poured into 500ml hydrochloric acid (2mol/L), and the reaction further underwent dichloromethane extraction (4 × 500ml), washing with water (3 × 500ml), anhydrous magnesium sulfate drying, column chromatography (dichloromethane/ethyl acetate = 100/1), solvent removal by vacuum distillation, and vacuum drying. A white solid (13.43g, yield 79.9%) was obtained. MS ESI+ 459$(M+H)^+$.

Step 4 Preparation of an intermediate compound 4 (shown below): N-[1-{[3-(aminomethyl)phenyl]methyl}-4-(methoxy)-1H-indazole-3-yl]-5-chloro-2-th iophene sulfonamide hydrochloride.

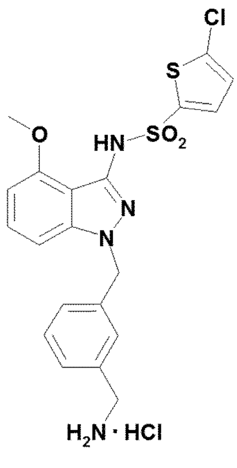

4

5-chloro-N-{1-[(3-cyanophenyl)methyl]-4-(methoxy)-1H-indazole-3-yl}-2-thiophene sulfonamide (15.75g, 34.4mmol) was dissolved in 200ml tetrahydrofuran and placed at 0°C. Under the protection of nitrogen, 1.0M lithium aluminum hydride solution in THF (86ml, 86mmol) was added dropwise into the reaction system, and the temperature of the system was kept below 10°C. After dropping, the system was transferred to room temperature and stirred to mix for 2h. A quenching reaction was carried out by adding 10ml water and 60ml 2mol/L sodium hydroxide solution. After stirring to mix for 30min, the solid was filtered out, the solid residue was washed with THF, and the filtrate was combined. The filtrate was further treated with a silica gel column, filtered and washed with methanol, and then washed with 10% 2N hydrochloric acid in 2.5L. A white solid (12.6g, yield 73.6%) was obtained after vacuum drying. MS ESI+ 463$(M+H)^+$.

Step 5 Preparation of an intermediate compound 5 (shown below): 2-{[(3-{[3-{[(5-chloro-2-thienyl)sulfonyl]amino}-4-(methoxy)-1H-indazole-1-yl]methyl }phenyl)methyl]amino}-1,1-dimethyl-2-oxoethyl acetate.

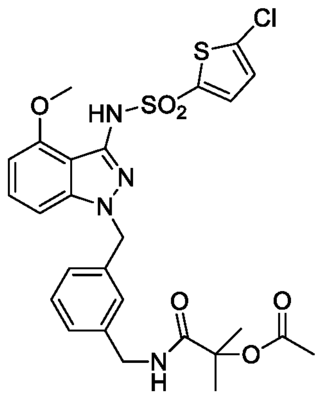

5

N-[1-{[3-(aminomethyl)phenyl]methyl}-4-(methoxy)-1H-indazole-3-yl]-5-chloro -2-thiophene sulfonamide hydrochloride (15.6 g, 31.3 mmol), triethylamine (9.6g, 95mmol) and dichloromethane (400ml) were mixed and stirred evenly in ice water bath. Then 2-chloro-1,1-dimethyl- 2-oxoethyl acetate (5.2g, 31.3mmol) was added dropwise. After dropping, stirring was continued for 2 hours at room temperature and under nitrogen protection. Washing was carried out with 2mol/L hydrochloric acid (150ml), saturated sodium bicarbonate solution (150ml) and saturated salt water (150ml), followed by anhydrous magnesium sulfate drying, column chromatography (petroleum ether/ethyl acetate =1/1), solvent removal by vacuum distillation, and vacuum drying to thereby obtain a white foam solid (14.1g, yield 76.3%). MS ESI+ 591$(M+H)^+$.

Step 6 Preparation of an intermediate compound 6 (shown below): N-[(3-{[3-{[(5-chloro-2-thienyl)sulfonyl]amino}-4-(methoxy)-1H-indazole-1-yl]methyl} phenyl)methyl]-2-hydroxy-2-methylpropionamide.

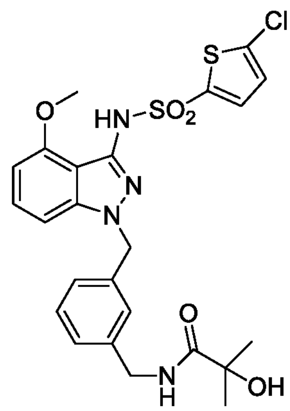

6

Potassium carbonate (4.6g, 33.3mmol) was added to methanol (350ml) solution containing 2-{[(3-{[3-{[(5-chloro-2-thienyl)sulfonyl]amino}-4-(methoxy)-1H-indazole-1-yl]methyl}phenyl)methyl]amino}-1,1-dimethyl-2-oxoethyl acetate (6.55g, 11.1mmol). After stirring for 4 h at room temperature, a large amount of solid was precipitated from the system. After the solvent is evaporated for removal by vaccum (i.e. solvent removal by vacuum distillation), 100ml water is added, and the pH is adjusted to acid with 2mol/L hydrochloric acid. Ethyl acetate was extracted (200ml × 2), and the extraction solution was washed with water (200ml × 2) until the water layer was neutral. The organic layer was dried with anhydrous magnesium sulfate. Then column chromatography (petroleum ether / ethyl acetate = 1 / 1.5), solvent removal by vacuum distillation, and vacuum drying were carried out to thereby obtain a white solid (5.08g, yield 83.5%). MS ESI+ 549$(M+H)^+$; 1H-NMR(400 MHz, DMSO-d6) : 10.40(s, 1H ), 8.13 (t, 1H), 7.33 (d, 1H ), 7.24-7.08 (m, 6H ), 6.93(d, 1H) , 6.46 (d, 1H), 5.45 (s, 2H), 5.33 (s, 1H), 4.17(d, 2H) , 3.72 (s, 3H), 1.20 (s, 6H).

Embodiment 2 Preparation of the compound of formula II: N-[(3-{[3-{[(5-chloro-2-thienyl)sulfonyl]amino}-4-(methoxy)-1H-indazole-1-yl]methyl} phenyl)methyl]-2-hydroxy-2-methylpropionamide sodium.

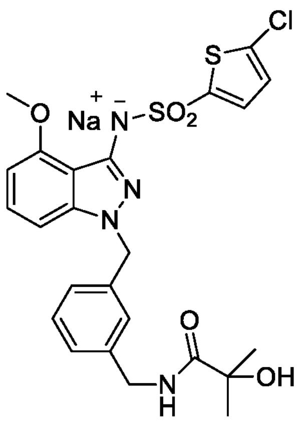

II

The compound of formula 6: N-[(3-{[3-{[(5-chloro-2-thienyl)sulfonyl] amino}-4-(methoxy)-1H-indazole-1-yl]methyl}phenyl)methyl]-2-hydroxy-2-methylpr opionamide (2.5g, 4.56mmol) was dissolved in 30ml of anhydrous tetrahydrofuran and stirred to mix until the solution was clear. NaH (60%) (182mg, 4.56mmol) was added into the system and stirred to mix for 1.0h at 35°C. A large amount of a white solid was produced in the system. The white solid was obtained by filtration, which was then washed with tetrahydrofuran and transferred to a vacuum drying oven for drying. A white solid (2.09g, yield 80.4%) was obtained. 1H-NMR (400 MHz, DMSO-d6) : 8.08 (t, 1H), 7.20 (d, 1H), 7.14-6.78 (m, 7H), 6.20 (d, 1H), 5.33 (s, 1H), 5.19 (s, 2H), 4.16(d, 2H), 3.74 (s, 3H), 1.21 (s, 6H).

Embodiment 3 Preparation of the compound of formula III: N-[(3-{[3-{[(5-chloro-2-thienyl)sulfonyl]amino}-4-(methoxy)-1H-indazole-1-yl]methyl} phenyl)methyl]-2-hydroxy-2-methylpropionamide potassium.

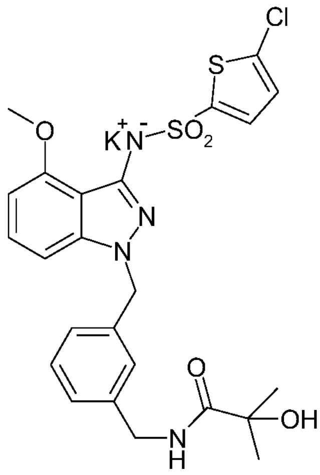

III

Method 1: The compound of formula 6: N-[(3-{[3-{[(5-chloro-2-thienyl)sulfonyl]amino}-4-(methoxy)-1H-indazole-1-yl]methyl}phenyl)meth yl]-2-hydroxy-2-methylpropionamide (2.5g, 4.56mmol) was dissolved in 30ml of anhydrous tetrahydrofuran and stirred to mix evenly until the solution was clear. KH (30%) (600mg, 4.56mmol) was added into the system and stirred to mix at 15°C for 3.5h. A large amount of a white solid was produced in the system. The white solid was obtained by filtration, washed with tetrahydrofuran, and transferred to a vacuum drying oven for drying. A white solid (2.21g, yield 83.1%) was then obtained. 1H-NMR (400 MHz, DMSO-d6) : 8.10 (t, 1H), 7.22 (d, 1H ), 7.17-6.79 (m, 7H ), 6.21 (d, 1H), 5.35 (s, 1H), 5.19 (s, 2H), 4.18(d, 2H) , 3.75 (s, 3H), 1.22 (s, 6H).

Method 2: The compound of formula 6: N-[(3-{[3-{[(5-chloro-2-thienyl)sulfonyl]amino}-4-(methoxy)-1H-indazole-1-yl]methyl}phenyl)meth yl]-2-hydroxy-2-methylpropionamide (1.37g, 2.5mmol) was dissolved in 15ml of anhydrous tetrahydrofuran and stirred to mix evenly until the solution was clear. Potassium tert-butoxide (280mg, 2.5 mmol) was added into the system and stirred to mix for 10min at room temperature. A large amount of a white solid was produced in the system. The white solid was obtained by filtration, washed with tetrahydrofuran, and then transferred to a vacuum drying oven for drying. A white solid (1.35g, yield 92.1%) was obtained. 1H-NMR (400 MHz, DMSO-d6): 8.09 (t, 1H),7.20 (d, 1H ), 7.13-6.78 (m, 7H), 6.20 (d, 1H), 5.34 (s, 1H), 5.18 (s, 2H ), 4.16(d, 2H), 3.74 (s, 3H), 1.21 (s, 6H).

Embodiment 4 Solubility tests.

Solubility of compounds was determined according to the method established in the general instructions of Part II of Pharmacopoeia 2010 edition. Respectively, 10, 100, and 1000 mg of compound 6 that was prepared in the Embodiment 1: N-[(3-{[3-{[(5-chloro-2-thienyl)sulfonyl]amino}-4-(methoxy)-1H-indazole-1-yl]methyl}phenyl)methyl]-2-hydroxy-2-methylpropionamide were accurately weighed, arranged in 500ml volumetric flasks at 25°C±2°C, and diluted with water to the scale, which were shaken vigorously for 30 seconds every 5 minutes. The dissolution was observed within 30 minutes. Only the sample added with 10mg of compound 6 showed no visible solute. The solubility in water was determined to be 0.02 mg/ml.

Respectively, 10, 100, 1000, and 2000 mg of the compound with formula II that was prepared in the Embodiment 2: N-[(3-{[3-{[(5-chloro-2-thienyl)sulfonyl] amino}-4-(methoxy)-1H-indazole-1-yl]methyl}phenyl)methyl]-2-hydroxy-2-methylpr opionamide sodium were accurately weighed, arranged in 2ml volumetric flasks at 25°C±2°C, and diluted with water to the scale, which were shaken vigorously for 30 seconds every 5 minutes. The dissolution was then observed within 30 minutes. No visible solute was observed for all samples except the 2000mg sample, and the solubility in water was determined to be 500 mg/ml.

Respectively, 8, 80, 800, and 1600 mg of the compound of formula III that was prepared by the method described in the Embodiment 3: N-[(3-{[3-{[(5-chloro-2-thienyl)sulfonyl]amino}-4-(methoxy)-1H-indazole-1-yl]methyl}phenyl)methyl]-2-hydroxy-2-methylpropionamide potassium were accurately weighed, arranged in 2ml volumetric flasks at 25°C±2°C, and diluted with water to the scale, which were shaken vigorously for 30 seconds every 5 minutes. The dissolution was then observed within 30 minutes. No visible solute was observed for all samples except the 800mg and

1600mg sample, and the solubility in water was determined to be 40 mg/ml.

The results are shown in Table 1.

Table 1

| Compound under test | Solubility in water under 25°C±2°C |
|---|---|
| Compound 6 | 0.02 mg/ml |
| Compound of formula II | 500 mg/ml |
| Compound of formula III | 40 mg/ml |

Compared with the above compound 6: N-[(3-{[3-{[(5-chloro-2-thienyl) sulfonyl]amino}-4-(methoxy)-1H-indazole-1-yl]methyl}phenyl)methyl]-2-hydroxy-2-methylpropionamide, compounds of formula II and formula III have significantly enhanced solubility under the same conditions. In an aqueous solution and at room temperature, the solubility of compounds of formula II and formula III is 500 mg/ml and 40 mg/ml, respectively, whereas under the same conditions, the solubility of N-[(3-{[3-{[(5-chloro-2-thienyl)sulfonyl]amino}-4-(methoxy)-1H-indazole-1-yl]methyl} phenyl)methyl]-2-hydroxy-2-methylpropionamide is 0.02 mg/ml. Thus the solubility is 2000-25000 times higher in relative terms.

Embodiment 5 *in vitro* CCR4 antagonistic activity tests.

The potency of antagonists was tested by a competitive test of [35S]-GTPyS. Briefly, CHO membranes expressing CCR4 were homogenized through a 23G needle. These membranes were then attached to WGA-coated Leadseeker SPA beads in a testing buffer (20mM HEPES, 10mM $MgCl_2$, 100mM NaCl, 0.05% BSA, 40ug/ml saponin, adjusted to pH 7.4 with KOH SM) to produce 3 μg/well final acceptance criteria (FAC) membrane and 250 μg/well FAC bead solution. After 60 minutes of pre-coupling on ice, GDP was added to obtain 4.4 uM FAC. Then [35S]-GTPyS, which was prepared in the testing buffer, was added into the bead/membrane solution to thereby obtain 0.33 nM FAC. Human MDC was added to the bead/membrane/[35S]-GTPyS suspension to obtain 80% (EC80) of FAC which showed the largest agonist response. The suspension of beads/membranes/[35S]-GTPyS/agonist was distributed into a white Greiner polypropylene 384-pore plate (45 μL/well), which contained 0.5 μl of compound. The final testing solution (45.5 μL) was sealed, centrifuged and incubated at room temperature for 3-6 hours. Then, the plate was read with Viewlux and the luminescence was plotted as the percentage of the maximum inhibitory response induced by $IC_{100}$ standard antagonist. The test results are shown in Table 2.

Table 2

| Compound under test | IC50 |
|---|---|
| Compound 6 | $3.54\times10^{-8}$mol/L |
| Compound of formula II | $7.47\times10^{-10}$mol/L |
| Compound of formula III | $2.26\times10^{-9}$mol/L |

Embodiment 6 *in vitro* membrane permeability test.

Caco-2 cell model is a human colonic adenocarcinoma cell line having a structure and function similar to differentiated intestinal epithelial cells. It has microvilli and other structures, and contains enzyme system related to intestinal brush border epithelium. It can be used to simulate intestinal transport *in vivo,* and has become a standard *in vitro* screening tool for predicting drug absorption in human small intestine and studying the drug transport mechanisms. The method to determine the drug absorption capacity can be $P_{app}$ (apparent permeability coefficient), with $P_{app} > 2 \times 10^{-6}$ determined to be a good-absorption drug.

1. Experimental materials and instruments

Caco-2 cells (gifted by School of Pharmacy, Zhejiang University), DMEM medium (GIBCO-BRL, USA), standard fetal bovine serum (FBS, Hydroxyne, USA), and nonessential amino acids (NEAA, Hyclone, USA), penicillin (Huabei Pharmaceutical Co., Ltd.), streptomycin (Huabei Pharmaceutical Co., Ltd.), HEPES (Amresco, USA), EDTA-Na (Beijing Chemical Plant), trypsin (1:250, Amresco, USA), glutamine (Sigma, USA), G418 (Merck, USA), and MilliPore ultra pure water.

Millicell insert-type culture dish (polycarbonate membrane, 0.4μm, 12mm diameter, MilliPore, USA), cell culture plate (COSTAR, USA), cell resistance meter (Millicell-ERS, MilliPore, USA), ultra clean bench (SW-CJ-IFD, Suzhou Antai Air Technology Co., Ltd.), carbon dioxide incubator (Sanyo, Japan), inverted microscope (CK, Olympus, Tokyo), culture dish (Corning, USA), ultra pure water machine (Simplicity, MilliPore, USA), fluorescence spectrophotometer (F-4500, Hitachi, Japan), electronic balance (BS 110S, Beijing Saiduolis Balance Co., Ltd.), LC-MS (Agilent 1100, LC-MSD_VL, DE, USA).

2. Experimental methods

2.1 Solution preparation

Preparation of culture medium: 10% FBS, 1% glutamine, 100 $U \cdot mL^{-1}$ penicillin and streptomycin double antibiotics solution, 1% nonessential amino acids, and 1.2 $mg \cdot L^{-1}$ G418 were added to DMEM when used.

Preparation of digestive solution: 1g trypsin and 80mg EDTA were added into 400ml phosphate buffer solution, which was further filtered with 0.22 μM filter membrane for sterilization, and was frozen storaged at -20°C for standby use.

Preparation of glutamine stock solution: glutamine 2.92g was added into 100ml PBS buffer, which was further filtered with 0.22 μM filter membrane for sterilization, sub-packaged in 1mL and frozen storaged at -20°C.

The preparation of penicillin stock solution: 0.8 million U of penicillin was added into 20ml of physiological saline; 1 million U of streptomycin was added into 25 ml physiological saline, which were mixed in 1:1, and was then filtered with 0.22 μM filter membrane for sterilization, sub-packaged in 1mL, and frozen storaged at -20°C.

HBSS solution preparation: NaCl 8.0g, KCl 0.4g, $Na_2HPO_4 \cdot H_2O$ 0.0475g, $KH_2PO_4$ 0.06g, and hapes 6g were added to ultrapure water to dissolve, the pH value was adjusted to 7.2-7.4, and water was added to a final 1L, which was then filtered with 0.22 μM filter membrane for sterilization, and storaged at -20°C.

2.2 Cell culture

The cryopreserved Caco-2 cells were thawed in 37°C water bath. After resuscitation, the cells were added into DMEM medium containing 10% FBS, and cultured in an incubator with 37°C, 5% $CO_2$ and 90% relative humidity. The culture medium was changed every other day. After 1-2 days of growth, the cells were digested with 0.25% trypsin-EDTA (0.2%) mixed digestive solution at 37°C and passaged in a certain proportion. The cell generation used in the experiments was 40-60 passages.

When cells reached 80% confluency, they were digested and suspended in complete medium and seeded on Millicell plate at $1 \times 10^6$ $cells \cdot ml^{-1}$. After that, the culture medium was changed once every 2 days and after 1 week, was changed once every 1 day. After 5 days of cluture, the resistance value reaches plateau (> 200 $\Omega \cdot cm^2$), which can be then used for membrane permeability experiment.

2.3 Quality control of Caco-2 monolayer cells:

2.3.1 Measurement of transmembrane epithelial cell electric resistance (TEER).

When measuring the transmembrane resistance, an electrode was immersed in DMEM culture medium for 24 hours, then it was taken out and immersed in 70% alcohol for disinfection for 15 minutes. After that, the electrode was placed at room temperature and dried naturally, and then put into sterile DMEM culture medium for 15 minutes. During the experiment, the two ends of the electrode were inserted into the upper and lower pools of each hole of the 24-well Millicell culture plate to detect the resistance value. The resistance value was measured three times at any point of each hole, and the resistance value of the blank hole was measured at the same time. The transmembrane resistance value (TEER) was calculated according to the following formula:

$$TEER = (R_t - R_0) \times S.$$

Herein, $R_t$ is the measured resistance; R0 is the resistance of the blank hole; and S is the effective film area.

2.3.2 Quality control with positive control compound:

Rho-123 was used as a positive control compound. The compound was diluted to 5 $\mu mol \cdot L^{-1}$ by HBSS. Before experiments, the culture medium in each well was discarded, washed twice with HBSS solution at 37°C, and then incubated in a 37°C incubator. Rho-123 was added in an upper pool, and HBSS solution was added in a lower pool,

and incubated in a constant-temperature shaker at 0, 30, 90, 120 min at each time point. Fluorescence spectrophotometer was used to detect the amount of Rho-123 in the lower pool. The emission wavelength was set at 430nm and the excitation wavelength was set at 530nm. The $P_{app}$ value of Rho-123 in this experiment is consistent with that reported in literature.

2.4 Drug membrane penetration test.

Before experiments, Millicell inoculated with cells was soaked in HBSS solution at 37°C for an appropriate time period, and the Millicell was washed slightly to remove the attachment on the cell surface. The permeability from cavity surface to basal surface: 0.35 ml HBSS solution containing drug was added to the top side (AP) and 1.2 ml blank HBSS solution was added to the bottom side (BL). The Millicell was incubated while shaking at 50 $r \cdot min^{-1}$ at 37°C, a 50μL sample was taken in the lower layer at 0, 30, 90 and 120 min, and a same volume of blank HBSS solution as added back. 3 holes were repeated for each concentration. 50 μL internal standard solution and 350 μL ethyl acetate were added to the sample, which was shaken and mixed, centrifuged at 12000rpm for 5min. 300 μL of supernatant was taken out, dried by evaporatation, and re-dissolved in 50μL acetonitrile. 10 μL sample was taken out for testing. For the permeability from the base surface to the cavity surface, the drug was added to the bottom side (BL), and the blank HBSS solution was added to the top side (AP). The following steps are the same as the permeability test operation from the cavity surface to the base surface.

The apparent permeability coefficient ($P_{app}$) of drugs reflects the ability of drugs to pass through monolayer cells and the speed and degree of drug absorption. It can be calculated by the following formula:

$$P_{app} = \frac{\Delta Q}{\Delta t \cdot A \cdot C_0}.$$

Herein, ΔQ is the amount of drug penetration during the time period of Δt, A is the cell surface area, which is the area of supporting membrane (0.6 $cm^2$) in this model, and $C_0$ is the initial concentration. The unit of $P_{app}$ is usually expressed in cm/h ($cm \cdot h^{-1}$) or cm/s ($cm \cdot s^{-1}$).

2.5 sample testing

LC/MS was used for testing. The standard curve (50nm-10000nM) was used to quantify the concentration of each sample.

3. Experimental results

| Compound under test | $P_{app}$ ( $\times 10^{-6}$ ) |
|---|---|
| Rho-123 | 4.89 |
| Compound 6 | 0.13 |
| Compound of formula II | 68.82 |
| Compound of formula III | 101.33 |

The inventor of the present application unexpectedly discovered and provided a compound of formula I as a CCR4 inhibitor, which has significantly enhanced solubility, better bioavailability and better *in vivo* and *in vitro* inhibitory activity of CCR4 compared with the prior art, which solves the issues that the existing CCR4 inhibitors have low solubility, affect the release of active pharmaceutical ingredients and causes low bioavailability, and fail to reach an expected blood drug concentration *in vivo* at a maximum safe dose.

The above is the description of the present disclosure, which shall not be regarded as a limitation of the invention provided therein. Unless otherwise pointed out, the implementation of the invention can use conventional technologies such as organic chemistry, polymer chemistry, biotechnology, etc. It is obvious that the invention can be realized in other ways besides the specific description in the above disclosure and embodiments. Other aspects and improvements within the scope of the disclosure will be apparent to those skilled in the art. Based on the teachings of the present disclosure, many changes and variations are feasible, as long as they are covered within the scope of the present disclosure.

Unless otherwise specified, the temperature unit "degree" in this present disclosure is referred to as Celsius degree, i.e., °C.

## Claims

1. A compound of formula I:

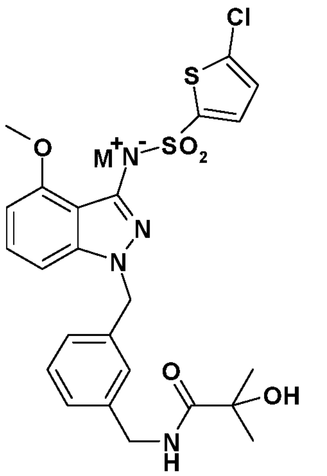

I

wherein M is Na or K.

2. A pharmaceutical composition, comprising the compound according to claim 1, and a pharmaceutically acceptable excipient mixed therewith.

3. Compound according to claim 1 for use in treating a CCR4-mediated disease, wherein the CCR4-mediated disease is asthma, chronic obstructive pulmonary disease (COPD) or rhinitis.

4. Use of the compound according to claim 1 in preparation of a medicine for treating a CCR4-mediated disease, wherein the CCR4-mediated disease is asthma, chronic obstructive pulmonary disease (COPD) or rhinitis.

## Patentansprüche

1. Verbindung der Formel I:

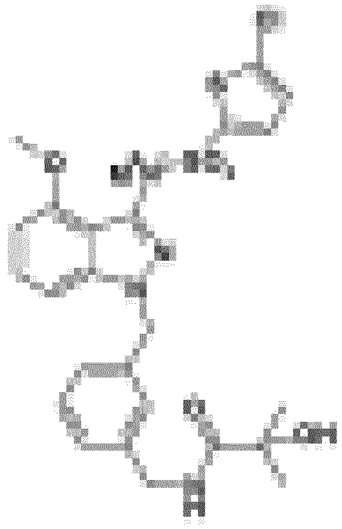

I

wobei M Na oder K ist.

2. Pharmazeutische Zusammensetzung, umfassend die Verbindung nach Anspruch 1 und einen damit vermischten pharmazeutisch akzeptablen Hilfsstoff.

3. Verbindung nach Anspruch 1 zur Verwendung bei der Behandlung einer CCR4-vermittelten Krankheit, wobei die CCR4-vermittelte Krankheit Asthma, chronisch obstruktive Lungenerkrankung (COPD) oder Rhinitis ist.

4. Verwendung der Verbindung nach Anspruch 1 zur Herstellung eines Medikaments zur Behandlung einer CCR4-vermittelten Krankheit, wobei die CCR4-vermittelte Krankheit Asthma, chronisch obstruktive Lungenerkrankung (COPD) oder Rhinitis ist.

## Revendications

1. Composé de formule I :

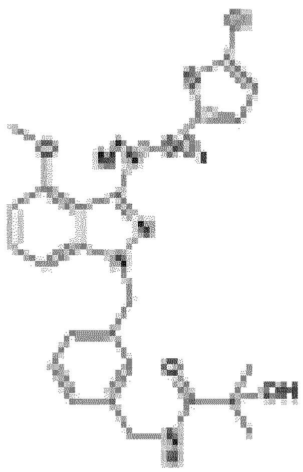

I

dans laquelle M représente Na ou K.

2. Composition pharmaceutique comprenant le composé selon la revendication 1, et un excipient pharmaceutiquement

acceptable mélangé avec celui-ci.

3. Composé selon la revendication 1, destiné à être utilisé dans le traitement d'une maladie médiée par CCR4, ladite maladie médiée par CCR4 étant l'asthme, la bronchopneumopathie chronique obstructive (BPCO) ou la rhinite.

4. Utilisation du composé selon la revendication 1 dans la préparation d'un médicament destiné au traitement d'une maladie médiée par CCR4, ladite maladie médiée par CCR4 étant l'asthme, la bronchopneumopathie chronique obstructive (BPCO) ou la rhinite.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201810032410
- CN 101370793 A
- CN 102388039 A


### Non-patent literature cited in the description


- **CHRISTINE AP et al.** *J. Biol. Chem.,* 1995, vol. 270 (8), 19495-19500
- **SADATOSHI MAEDA et al.** *Veterinary Immunology and Immunology,* 2002, (90), 145-154
- **HAN WL et al.** *Biochem J.,* 2001, vol. 357, 127-135
- Pharmacopoeia. 2010